**Europäisches Patentamt**

**European Patent Office**

**Office européen des brevets**

(11) Numéro de publication : **0 461 171 B1**

(12) **FASCICULE DE BREVET EUROPEEN**

(45) Date de publication du fascicule du brevet :
**14.04.93 Bulletin 93/15**

(21) Numéro de dépôt : **90904367.1**

(22) Date de dépôt : **26.02.90**

(86) Numéro de dépôt international :
**PCT/FR90/00132**

(87) Numéro de publication internationale :
**WO 90/09845 07.09.90 Gazette 90/21**

(51) Int. Cl.⁵ : **B01J 29/18**, C10G 45/64, C07C 5/27

(51) Int. Cl.⁵ : $B01J\ 29/18$, $C10G\ 45/64$, $C07C\ 5/27$

(54) **CATALYSEUR A BASE DE MORDENITE RENFERMANT AU MOINS UN METAL DES GROUPES IIA, IVB, IIB OU IVA ET SON UTILISATION EN ISOMERISATION D'UNE COUPE C8 AROMATIQUE.**

Le dossier contient des informations techniques présentées postérieurement au dépôt de la demande et ne figurant pas dans le présent fascicule.

(30) Priorité : **03.03.89 FR 8902946**

(43) Date de publication de la demande :
**18.12.91 Bulletin 91/51**

(45) Mention de la délivrance du brevet :
**14.04.93 Bulletin 93/15**

(84) Etats contractants désignés :
**BE DE FR GB IT NL**

(56) Documents cités :
**EP-A- 0 234 974**
**EP-A- 0 253 743**
**FR-A- 2 477 903**
**US-A- 3 751 502**

(73) Titulaire : **INSTITUT FRANCAIS DU PETROLE
4, Avenue de Bois Préau
F-92502 Rueil-Malmaison (FR)**

(72) Inventeur : **BASSET, Jean-Marie
21, petite-rue de la Dova
F-69100 Villeurbanne (FR)**
Inventeur : **CHOPLIN, Agnès
46, rue du Docteur-Ollier
F-69100 Villeurbanne (FR)**
Inventeur : **RAATZ, Francis
10, allée Jacques-Prévert
F-78260 Achères (FR)**
Inventeur : **THEOLIER, Albert
42bis, rue Raspail
F-69150 Décines (FR)**
Inventeur : **TRAVERS, Christine
25 bis rue des Coudreaux
F-92500 Rueil-Malmaison (FR)**

(74) Mandataire : **Andreeff, François et al
INSTITUT FRANCAIS DU PETROLE 4, avenue
de Bois-Préau
F-92502 Rueil-Malmaison (FR)**

**Description**

La présente invention concerne un catalyseur de type alumino-silicate comprenant une mordénite forme H dont on a modifié la sélectivité géométrique par dépôt selectif sur la surface extérieure de ses cristaux d'au moins un métal choisi parmi les métaux des groupes IIa (Be, Mg, Ca, Sr, Ba, Ra), IVb (Ti, Zr, Hf), IIb (Zn, Cd, Hg) et IVa (Ge, Sn, Pb) de la classification périodique des éléments (Handbook of Chemistry and Physics, 61ème édition, 1980-81), éventuellement au moins un métal du groupe VIII de ladite classification et une matrice et son utilisation dans les réactions d'isomérisation des hydrocarbures C8 aromatiques. Elle concerne également le procédé de préparation de ladite mordénite.

Actuellement les catalyseurs utilisés industriellement dans ces réactions sont essentiellement à base de zéolithe ZSM5, seule ou en mélange avec d'autres zéolithes, telles que la mordénite par exemple. Ces catalyseurs sont notamment décrits dans les brevets US-A-4467129, 4482773 et EP-B-0138617.

L'intérêt de la zéolithe ZSM5 réside dans son excellente sélectivité de forme, qui conduit à une grande sélectivité en paraxylène, la sélectivité vis-à-vis des réactions secondaires indésirables de dismutation restant à un niveau très faible. La mordénite est une zéolithe très active pour la réaction d'isomérisation des C8 aromatiques, en particulier plus active que la ZSM5, cependant elle ne possède pas de propriétés de sélectivité géométrique particulières. Ceci se traduit, quelque soit son rapport Si/Al, par des sélectivités en paraxylène plus faibles que celles obtenues pour la zéolithe MFI et surtout par des productions de triméthylbenzènes très importantes. La production de triméthylbenzènes par dismutation est en effet favorisée dans la mordénite dont le système microporeux est plus ouvert que celui de la ZSM5 : les ouvertures sont à 12 oxygènes au lieu de 10 pour la ZSM5.

La demanderesse a découvert qu'il est possible, grâce au dépôt sur la surface extérieure des cristaux de mordénite d'au moins un métal choisi parmi les métaux des groupes IIa, IVb, IIb et IVa et en particulier de magnésium, de titane, de zinc ou/et d'étain, d'obtenir des catalyseurs actifs et sélectifs pour la réaction d'isomérisation des C8 aromatiques. Cette procédure de préparation nouvelle confère à la mordénite des propriétés de sélectivité géométrique très améliorées, ce qui se traduit par une inhibition spectaculaire des réactions secondaires indésirables telles que la dismutation. Cette nouvelle mordénite modifiée conduit par ailleurs à des sélectivités vis-à-vis des réactions parasites de désalkylation inférieures à celles des catalyseurs basés sur la ZSM5. On aboutit ainsi à des solides qui présentent des performances en isomérisation des C8 aromatiques non seulement meilleures que celles des mordénites de l'art antérieur, mais également au moins équivalentes, voire supérieures, aux performances des catalyseurs à base de ZSM5.

La mordénite utilisée dans le catalyseur de la présente invention est fabriquée à partir d'une mordénite soit du type à petits pores soit du type à larges pores.

La mordénite du type à petits pores possède une teneur pondérale en sodium par rapport au poids de mordénite sèche généralement comprise entre 4 et 6,5 %, un rapport atomique Si/Al global généralement compris entre 4,5 et 6,5, un volume de maille élémentaire généralement compris entre 2,76 et 2,80 nm³ (avec 1 nm = $10^{-9}$ m) et n'adsorbe habituellement que des molécules de diamètre cinétique inférieur à environ $4{,}4 \times 10^{-10}$ m.

La mordénite du type à larges pores se distingue de celle du type à petits pores par le fait qu'elle peut adsorber des molécules de diamètre cinétique supérieur à environ $6{,}6 \times 10^{-10}$ m, donc en particulier les molécules de benzène, que son rapport atomique Si/Al global est généralement compris entre 4,5 et 20 et que son volume de maille élémentaire est habituellement compris entre 2,74 et 2,79 nm³.

Avant de sélectiver le réseau poreux de la mordénite par dépôt d'au moins un métal des groupes IIa, IVb, IIb, IVa, il est préférable de préparer tout d'abord la forme H de ladite mordénite avec une teneur pondérale en sodium par rapport au poids de mordénite sèche généralement inférieure à 2000 ppm, de préférence à 1000 ppm et, de manière encore plus préférée, à 500 ppm, un rapport atomique Si/Al global compris entre 6 et 15, un volume de maille élémentaire compris entre 2,725 et 2,785 nm³, une capacité d'adsorption de n-hexane mesurée à 0°C sous une pression de 13 Torr ($1{,}7 \times 10^3$ Pa) supérieure à 0,065 cm³ liquide/gramme (cm³ de n-hexane liquide par gramme de mordénite) et une capacité d'adsorption d'isooctane mesurée à 40°C sous une pression de 26 Torr ($3{,}4 \times 10^3$ pa) supérieure à 0,068 cm³ liquide/gramme. La teneur pondérale en eau de la mordénite forme H est habituellement comprise entre 5 et 40 % et, de préférence, entre 10 et 30 %.

Pour cela, on pourra employer toute technique connue de l'homme du métier comme, par exemple, l'attaque acide directe, la calcination en présence ou non de vapeur d'eau de la forme $NH_4^+$ suivie d'une ou de plusieurs attaques acides, les cycles "calcination(s) - attaque(s) acide(s)", les traitements par les fluorosilicates ou par $SiCl_4$ etc. Dans le cas spécifique de la mordénite à petits pores, il faut s'assurer que les traitements retenus ont bien conduit à une ouverture des canaux.

Le dépôt du ou des métaux choisis parmi les métaux des groupes IIa, IVb, IIb et IVa est réalisé avantageusement par greffage en utilisant comme agent de greffage au moins un composé organométallique dudit métal qui est, d'une part, suffisamment volumineux pour ne pas pénétrer à l'intérieur du réseau microporeux de la mordénite forme H et, d'autre part, susceptible de réagir avec les groupes OH de surface. On pourra notamment employer comme agents de greffage, dans le cas du magnésium, les composés de formule $MgR^1R^2$ (organomagnésiens) dans lesquels les groupes $R^1$ et $R^2$, identiques ou différents, sont des groupes organiques d'encombrement varié et, en général, d'encombrement important ; à titre d'exemples non limitatifs, on peut citer les radicaux alkyl, aryl, organosilyl vinyl, polynucléaire aryl, cyclo-alkyl, allyl, proporgyl. Lorsque le groupe $R^1$ est un des groupes cités précédemment, le groupe $R^2$ peut également être un groupe de type alkoxy ou aryl-oxy encombré, comme par exemple un groupe ter-butoxy, o,o'-diphényl-phénoxy, o,o'-di-isopropyl phénoxy.

Dans le cas des autres métaux du groupe IIa (Be, Ca, Sr, Ba, Ra) et des métaux des groupes IVb (Ti, Zr, Hf), IIb (Zn, Cd, Hg) et IVa (Ge, Sn, Pb), on pourra utiliser, comme agents de greffage, également des composés de formule $BeR_2$, $CaR_2$, $SrR_2$, $BaR_2$, $RaR_2$, $TiR_4$, $ZrR_4$, $HfR_4$, $CdR_2$, $HgR_2$, $GeR_4$, $SnR_4$ ou $PbR_4$ dans lesquels R, identiques ou différents entre eux, sont des groupes organiques tels que ceux définis précédemment dans le cas de $MgR^1R^2$.

Dans le cas du magnésium, un agent de greffage préféré est le bisnéopentylmagnésium noté $Mg(Np)_2$. Généralement après une mise sous vide ou sous gaz inerte de la mordénite à une température supérieure à 150°C, de préférence entre 225 et 525° C, par exemple à 400° C, pendant 2,5 à 5,5 heures, par exemple pendant 4 heures (étape de séchage), puis une diminution de la température sous vide ou sous gaz inerte jusqu'à une température comprise entre 100 et 200°C, de préférence égale à 150°C (pour la voie phase gazeuse définie plus loin) ou jusqu'à une température comprise entre 5 et 35°C, de préférence égale à 20°C (pour la voie phase liquide définie plus loin), l'agent de greffage peut être greffé sur la mordénite par la voie phase gazeuse (sublimation de $Mg(Np)_2$) ou par la voie phase liquide ($Mg(Np)_2$ en solution dans un solvant, par exemple l'éther, sous vide ou sous gaz inerte). Par la voie phase gazeuse, la température de greffage (température à laquelle s'effectue le greffage) est avantageusement supérieure à 50°C et, de préférence, comprise entre 100 et 200°C; par la voie phase liquide, ladite température est avantageusement supérieure à la température de congélation du solvant utilisé (-116° C dans le cas de l'éther par exemple) et, de préférence, comprise entre 5 et 35°C. Les durées de traitement (greffage) sont habituellement supérieures à 1 minute et sont avantageusement comprises entre 10 et 60 minutes. Après greffage, l'excès de $Mg(Np)_2$ présent dans le système est éliminé généralement par une purge sous vide ou sous gaz inerte dans le cas de la voie phase gazeuse ; dans le cas de la voie phase liquide, on préfère éliminer d'abord la phase liquide, sous vide ou sous gaz inerte, puis l'excès de $Mg(Np)_2$ par un rinçage (ou lavage) avec un solvant (tel que celui employé précédemment).

Après cette élimination de l'excès de réactif, la mordénite subit habituellement un traitement thermique destiné à décomposer les fragments organiques Np liés aux atomes de magnésium fixés sur la surface extérieure. Ce traitement est avantageusement réalisé en présence d'oxygène (de préférence en mélange avec un gaz inerte tel que l'azote) à une température supérieure à environ 250°C, de préférence à environ 350°C et, de manière plus avantageuse, comprise entre 350 et 600°C.

Le dépôt éventuel d'autres métaux des groupes IIa, IVb, IIb ou IVa à la place ou en plus du magnésium s'effectue de manière analogue à celui décrit précédemment pour le magnésium en utilisant un agent de greffage adéquat, tel qu'un composé organométallique.

A l'issue du traitement thermique, la teneur pondérale de la mordénité en métal du groupe IIa, IVb, IIb ou/et IVa (par exemple en magnésium) est de préférence comprise entre 0,05 et 0,30 % et, de manière avantageuse, entre 0,10 et 0,25 %.

Néanmoins, on peut éventuellement régler le niveau de sélectivité de la mordénite en procédant au besoin à un ou plusieurs cycles supplémentaires "greffage de métal (métaux) des groupes IIa, IVa, IIb ou IVb-calcination", suivant la technique décrite précédemment, de manière à atteindre des teneurs pondérales en métal (métaux) desdits groupes (par exemple en magnésium) supérieures à 0,30 % et, si nécessaire, à 1,0 %.

Les propriétés acides de la mordénite ne sont pas altérées par le dépôt dudit (desdits) métal (métaux) selon la technique décrite ci-dessus.

Ainsi, la mordénite obtenue possède une teneur pondérale en sodium par rapport au poids de mordénite sèche inférieure à 2000 ppm, de préférence à 1000 ppm et, de manière encore plus préférée, à 500 ppm, un rapport atomique Si/Al global compris entre 6 et 15 et, de préférence, entre 7,5 et 13,5 et un volume de maille élémentaire compris entre 2,725 et 2,785 $nm^3$.

En revanche, sa capacité d'adsorption pour les hydrocarbures encombrés ou ramifiés (isooctane par exemple) est fortement diminuée alors que sa capacité d'adsorption pour les hydrocarbures linéaires (n-hexane par exemple) n'est pas affectée. Ainsi, sa capacité d'adsorption de n-hexane mesurée à 0°C sous une pression de 13 Torr ($1,7 \times 10^3$ Pa) est supérieure à 0,065 $cm^3$ liquide/gramme et, de préférence, supérieure à 0,072 $cm^3$ liquide/gramme ; sa capacité d'adsorption d'isooctane mesurée à 40°C sous une pression de 26 Torr ($3,4 \times 10^3$ Pa) est inférieure à 0,068 $cm^3$ liquide/gramme et, de préférence, inférieure à 0,058 $cm^3$ liquide/gramme. Cette capacité d'adsorption limitée pour les hydrocarbures ramifiés, tels que l'isooctane, mesurée à basse température ne signifie pas que les C8 aromatiques ne pénètrent pas dans le volume poreux de la mordénite lors de la réaction d'isomérisation à haute température. En fait, elle indique qu'il y aura une limitation diffusionnelle sévère pour les triméthylbenzènes à haute température.

Les caractéristiques de la mordénite peuvent être mesurées par les méthodes suivantes :
- les capacités d'adsorption sont établies par gravimétrie,
- les rapports atomiques Si/Al sont déterminés par spectrométrie infra-rouge, les teneurs en sodium par adsorption atomique,
- le volume de maille élémentaire est déterminé par diffraction X, l'échantillon de mordénite étant préparé comme dans le mode opératoire de la norme ASTM D 3942 80 établie pour la faujasite.

La mordénite peut être soumise (avant ou après le dépôt d'au moins un métal choisi parmi les métaux des groupes IIa, IVb, IIb et IVa) au dépôt d'au moins un métal du groupe VIII, de préférence choisi dans le groupe formé par le platine et le palladium, et mise en forme par toute technique connue de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière. La teneur en mordénite du support (mordénite + matrice) ainsi obtenu est généralement comprise entre environ 0,5 et 99,99 % et avantageusement comprise entre environ 40 et 90 % en poids par rapport au support. Elle est plus particulièrement comprise entre environ 60 et 85 % en poids par rapport au support. La teneur en matrice du catalyseur est généralement comprise entre environ 0,01 et 99,5 %, avantageusement entre environ 10 et 60 % et, de préférence, entre environ 15 et 40 % en poids par rapport au support (mordénite + matrice).

La mise en forme peut être réalisée avec d'autres matrices que l'alumine, telles que, par exemple, la magnésie, la silice-alumine, les argiles naturelles (kaolin, bentonite) et par d'autres techniques que l'extrusion, telles que le pastillage ou la dragéification.

Le métal hydrogénant du groupe VIII, de préférence Pt et/ou Pd, est ensuite déposé sur le support par tout procédé connu de l'homme de l'art et permettant le dépôt du métal sur la mordénite. On peut utiliser la technique d'échange cationique avec compétition, où l'agent compétiteur est de préférence le nitrate d'ammonium, le rapport de compétition étant au moins égal à environ 50 et avantageusement d'environ 50 à 200. Dans le cas du platine ou du palladium, on utilise habituellement un complexe tétrammine du platine ou un complexe tétrammine du palladium ; ces derniers se déposeront alors pratiquement en totalité sur la mordénite. Cette technique d'échange cationique peut également être utilisée pour déposer directement le métal sur la poudre de mordénite, avant son mélange éventuel avec une matrice.

Le dépôt du métal (ou des métaux) du groupe VIII est suivi en général d'une calcination sous air ou oxygène, usuellement entre 300 et 600°C durant 0,5 à 10 heures, de préférence entre 350 et 550°C durant 1 à 4 heures. On peut procéder ensuite à une réduction sous hydrogène, généralement à une température comprise entre 300 et 600°C pendant 1 à 10 heures ; de préférence, on opérera entre 350 et 550°C pendant 2 à 5 heures. La teneur en métal du groupe VIII (de préférence Pt et/ou Pd) déposé sur le catalyseur et obtenue à l'issue de l'échange est comprise habituellement entre 0,05 et 1,5 %, de préférence entre 0,1 et 1 %, en poids par rapport à l'ensemble du catalyseur.

On peut également déposer le platine et/ou le palladium non plus directement sur la mordénite, mais sur le liant aluminique, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. En général, après le dépôt de platine et/ou de palladium, le catalyseur est comme précédemment soumis à une calcination puis réduit sous hydrogène comme indiqué ci-dessus.

Le catalyseur bifonctionnel obtenu par les procédures précédentes peut être mis en oeuvre notamment dans les réactions d'isomérisation d'une coupe C8 aromatique, comprenant par exemple soit uniquement un mélange de xylènes, soit un mélange de xylène(s) et d'éthylbenzène. L'isomérisation des alkyl-aromatiques, et en particulier des xylènes, revêt une importance commerciale considérable. C'est en général surtout le paraxylène qui est le produit le plus recherché, car il est notamment utilisé comme intermédiaire dans la fabrication des fibres polyesters. On préfère fabriquer le paraxylène en isomérisant le métaxylène, qui lui peut être obtenu par isomérisation de l'orthoxylène. L'éthylbenzène, qui est difficilement séparable par distillation du mélange des xylènes (les points d'ébullition des différents composés sont très proches), se trouve très souvent dans la charge d'isomérisation des hydrocarbures aromatiques en C8.

Les conditions opératoires du procédé d'isomérisation d'une coupe C8 aromatique effectué en présence d'au moins un catalyseur selon l'invention sont habituellement les suivantes :
- température comprise entre 240 et 600°C, de préférence entre 350 et 510°C,
- pression comprise entre 0,5 et 100 bars, de préférence entre 2 et 30 bars,
- vitesse spatiale (pph), en masse de charge par unité de charge de catalyseur et par heure, comprise entre 0,5 et 200, de préférence entre 2 et 100,

- rapport molaire hydrogène sur hydrocarbures de la charge (H₂/HC) compris entre 0,5 et 12, de préférence entre 2 et 6.

Les exemples qui suivent illustrent l'invention sans toutefois en limiter la portée ; ils sont donnés pour une charge formée de 75 % d'orthoxylène et de 25 % d'éthylbenzène (% en poids).

EXEMPLE 1 : Catalyseurs A1 et A2 conformes à l'invention.

La matière première utilisée est une mordénite "larges pores ", référencée Zéolon 100 Na de la société Norton ; elle possède un rapport atomique Si/Al global égal à 6,0, une teneur pondérale en sodium par rapport au poids de mordénite sèche d'environ 4,5 %, un volume de maille élémentaire de 2,780 nm³ ; elle peut absorber de plus des molécules de diamètre cinétique supérieur à environ 6,6x10⁻¹⁰m.

Cette mordénite subit tout d'abord un échange ionique dans une solution de NH₄NO₃ 10N à environ 100°C pendant 4 heures ; puis, le solide obtenu est soumis à trois attaques acides à l'aide d'acide chlorhydrique 0,5N, à environ 20°C, pendant 4 heures, le volume V de la solution d'acide chlorhydrique engagée étant égal à 20 fois le poids de mordénite sèche (V/P = 20 cm³/g).

A l'issue de ces traitements, la mordénite sous forme H a un rapport atomique Si/Al global égal à 9,0, une teneur pondérale en sodium par rapport au poids de mordénite sèche de 350 ppm, un volume de maille élémentaire de 2,762 nm³, une capacité d'adsorption de n-hexane (à 0°C, sous 13 Torr (1,7x10³Pa)) de 0,075 cm³ liquide/g et une capacité d'adsorption d'isooctane (à 40°C, sous 26 Torr (3,4x10³pa)) de 0,070 cm³ liquide/g.

On dépose ensuite du magnésium sur la surface externe des cristaux de la mordénite forme H par la méthode en phase gazeuse (i) et par la méthode en phase liquide (ii).

i) Méthode en phase gazeuse.

On opère selon les étapes successives suivantes :
- mise sous vide de la mordénite à 400°C pendant 4 heures,
- descente sous vide jusqu'à 150°C,
- mise en contact de la mordénité pendant 30 minutes avec un réservoir porté à 150°C contenant Mg(Np)₂ (étape de sublimation),
- isolement de la mordénité et élimination de l'excès de Mg(Np)₂ par pompage sous vide à 150°C,
- montée à 450°C sous un mélange 95 % N₂ + 5 % O₂, puis passage sous air pur à 450°C et maintien sous air à cette température pendant 2 heures, descente sous air jusqu'à température ambiante (environ 20° C).

Le solide obtenu à l'issue de ces traitements est référencé HM1 : sa teneur pondérale en magnésium est de 0,16 %, sa capacité d'adsorption de n-hexane (à 0°C, sous 13 Torr (1,7x10³ Pa)) de 0,075 cm³ liquide/g et sa capacité d'adsorption d'isooctane (à 40°C, sous 26 Torr (3,4x10³ Pa)) de 0,038 cm³ liquide/g, ses autres caractéristiques restant inchangées par rapport à celles de la mordénite forme H.

ii) Méthode en phase liquide

On opère selon les étapes successives suivantes :
- mise sous vide de la mordénité à 400°C pendant 4 heures,
- descente sous vide jusqu'à 20°C,
- mise en contact de la mordénite sous argon pendant 25 minutes à 20°C avec une solution de Mg(Np)₂ dans l'éther,
- élimination sous argon de la phase liquide,
- lavage par une solution d'éther fraîche,
- montée à 450°C sous un mélange 95 % N₂ + 5 % O₂, puis passage sous air pur à 450°C et maintien sous air à cette température pendant 2 heures, descente sous air jusqu'à température ambiante (environ 20° C).

Le solide obtenu à l'issue de ces traitements est référencé HM2 : sa teneur pondérale en magnésium est de 0,15 %, sa capacité d'adsorption de n-hexane (à 0°C, sous 13 Torr (1,7x10³ pa)) de 0,074 cm³ liquide/g et sa capacité d'adsorption d'isooctane (à 40°C, sous 26 Torr (3,4x10³ Pa)) de 0,039 cm³ liquide/g, ses autres caractéristiques étant inchangées par rapport à celles de la mordénite forme H.

Les deux solides HM1 et HM2 subissent ensuite les mêmes traitements : ils sont chacun mélangés intimement avec de l'alumine sur laquelle on a dispersé 0,3 % en poids de platine, le support constitué par le mélange mordénite HM1-alumine (ou mordénite HM2-alumine) contenant 39 % en poids d'alumine. La teneur pondérale en platine de chacun des catalyseurs finaux A1 (contenant HM1) et A2 (contenant HM2) est donc d'environ 0,12 %.

Les catalyseurs ainsi fabriqués sont ensuite mis en forme par pastillage, calcinés sous air à 500°C durant 2 heures et réduits sous hydrogène à 500°C pendant 3 heures.

Ces catalyseurs A1 et A2 sont alors testés en isomérisation du mélange orthoxylène (75 % poids) et éthylbenzène (25 % poids), à une température de 420°C, sous une pression de 15 bars, avec une vitesse spatiale (pph) de 50 (heure)$^{-1}$ et un rapport molaire hydrogène sur hydrocarbures ($H_2$/HC) de 4 environ.

Les performances des catalyseurs A1 et A2 (et des catalyseurs préparés dans les exemples suivants), reportées dans le tableau 1, sont définies par :

$$\text{Conversion de l'o-xylène(\%)} = \left(\frac{\text{Masse d'o-xylène dans la charge-masse d'o-xylène dans la recette}}{\text{Masse d'o-xylène dans la charge}}\right) \times 100$$

$$\text{Sélectivité en isomérisation (\%)} = \left(\frac{\text{Masse de m-xylène + masse p-xylène}}{\text{Masse des produits}}\right) \times 100$$

$$\text{Rendement en isomérisation (\%)} = \frac{\text{Conversion} \times \text{sélectivité}}{100}$$

$$\text{Sélectivité en dismutation (\%)} = \left(\frac{\text{Masse de triméthylbenzène+masse de toluène+masse de benzène}}{\text{Masse des produits}}\right) \times 100$$

$$\text{Sélectivité en craquage (\%)} = \frac{\text{Masse des gaz de C1 à C4}}{\text{Masse des produits}} \times 100$$

EXEMPLE 2 : Catalyseur B conforme à l'invention.

La matière première utilisée est une mordénite "petits pores", référencée Alite 150 de la Société Chimique de la Grande Paroisse. Sa formule chimique à l'état anhydre est : Na $AlO_2(SiO_2)_{5,5}$, sa teneur pondérale en sodium par rapport au poids de mordénite sèche est d'environ 5,3 %, son volume de maille élémentaire de 2,790 nm$^3$ ; elle n'adsorbe que des molécules de diamètre au plus égal à 3,8x10$^{-10}$ m ; 50 grammes de cette poudre sont plongés dans une solution 2M de nitrate d'ammonium et la suspension est portée à 95°C pendant 2 heures.

Le volume de la solution de nitrate d'ammonium engagée est égal à 4 fois le poids de mordénite sèche (V/P = 4 cm$^3$/g). Cette opération d'échange cationique est recommencée 3 fois. Après le 3ème échange, le produit est lavé à l'eau, à 20°C pendant 20 minutes, avec un rapport V/P égal à 4. La teneur en sodium, exprimée en pourcentage en poids par rapport au poids de solide sec, passe de 5,5 à 0,1 %. Le produit est ensuite filtré et soumis à une calcination en atmosphère confinée ("self-steaming") à 600°C pendant 2 heures (l'atmosphère de calcination contenant au moins 5 % de vapeur d'eau).

On procède ensuite à une attaque acide : le solide est porté à reflux dans une solution aqueuse d'acide chlorhydrique 0,58N à 90°C pendant 2 heures, avec un rapport V/P égal à 8 cm$^3$/g (où V est le volume de la solution d'acide chlorhydrique et P le poids de mordénite sèche). Le produit est ensuite filtré, lavé à l'acide chlorhydrique 0,1N puis à l'eau.

La mordénite forme H ainsi obtenue a un rapport atomique Si/Al global égal à 12, une teneur pondérale en sodium par rapport au poids de mordénite sèche de 300 ppm, un volume de maille élémentaire de 2,750 nm$^3$, une capacité d'adsorption de n-hexane (à 0°C, sous 13 Torr (1,7x10$^3$ Pa)) de 0,075 cm$^3$ liquide/g et une capacité d'adsorption d'isooctane (à 40°C, sous 26 Torr (3,4x10³ Pa)) de 0,071 cm³ liquide/g.

On dépose ensuite du magnésium sur la surface externe des cristaux de ladite mordénite forme H suivant la méthode en phase liquide décrite dans l'exemple 1. La mordénite obtenue possède alors une teneur pondérale en magnésium de 0,17 %, une capacité d'adsorption de n-hexane (à 0°C, sous 13 Torr (1,7x10³ Pa)) de 0,075 cm³ liquide/g et une capacité d'adsorption d'isooctane (à 40°C, sous 26 Torr (3,4x10³ Pa)) de 0,039 cm³ liquide/g, ses autres caractéristiques restant inchangées par rapport à celles de la mordénite forme H.

Les étapes de mélange mordénite-alumine, de dispersion du platine, de mise en forme, de réduction du catalyseur et les conditions de test en isomérisation sont identiques à celles décrites dans l'exemple 1.

Les performances du catalyseur B ainsi obtenu (dont la teneur pondérale en platine est d'environ 0,12 %) sont reportées dans le tableau I.

EXEMPLE 3 : Catalyseur C non conforme à l'invention.

Le catalyseur C diffère des catalyseurs A1 et A2 en ce que la mordénite forme H, obtenue à partir de la mordénite "larges pores" référencée Zéolon 100 Na, ne subit pas de dépôt de magnésium.

Les étapes de mélange mordénite-alumine, de dispersion du platine, de mise en forme, de réduction du catalyseur et les conditions de test en isomérisation sont identiques à celles décrites dans l'exemple 1.

Les performances du catalyseur C ainsi obtenu (dont la teneur pondérale en platine est d'environ 0,12 %) sont reportées dans le tableau I.

EXEMPLE 4 : Catalyseur D non conforme à l'invention.

Le catalyseur D renferme une zéolithe de structure MFI, synthétisée en milieu fluorure ; cette zéolithe possède un rapport atomique Si/Al global de 250. Sa teneur pondérale en sodium est de 50 ppm par rapport au poids de zéolithe sèche. Son volume de maille élémentaire est égal à 5,360 nm³. Sa capacité d'adsorption de n-hexane (à 0°C, sous 13 Torr (1,7x10³ Pa)) est de 0,071 cm³ liquide/g et sa capacité d'adsorption d'isooctane (à 40°C, sous 26 Torr (3,4x10³ Pa)) de 0,028 cm³ liquide/g.

Les étapes de mélange zéolithe-alumine, de dispersion du platine, de mise en forme, de calcination, de réduction du catalyseur et les conditions de test en isomérisation sont identiques à celles décrites dans l'exemple 1, mais avec une pph de 30 (heures)⁻¹.

Les performances du catalyseur D ainsi obtenu (dont la teneur pondérale en platine est d'environ 0,12 %) sont reportées dans le tableau I.

Les catalyseurs A1, A2 et B conformes à l'invention sont plus performants que les catalyseurs C et D de l'art antérieur : le rendement en isomérisation des C8 aromatiques des catalyseurs A1, A2 et B est nettement supérieur à celui des catalyseurs C et D. Le catalyseur D est ainsi beaucoup moins actif que les catalyseurs A1, A2 et B et le catalyseur C est très peu sélectif ; en particulier, avec les mordénites sélectivées selon l'invention (catalyseurs A1, A2 et B), la réaction secondaire de dismutation conduisant à la formation de triméthylbenzènes est très fortement inhibée par rapport à ce qu'on obtient en présence de mordénite non sélectivée (catalyseur C).

## TABLEAU I

| Catalyseur | A1 | A2 | B | C | D |
|---|---|---|---|---|---|
| Exemple | 1 | 1 | 2 | 3 | 4 |
| pph ($h^{-1}$) | 50 | 50 | 50 | 50 | 30 |
| Conversion (%) de l'o-xylène | 70,5 | 71,0 | 71,0 | 79,5 | 50,0 |
| Sélectivité en isomérisation(%) | 80,1 | 80,2 | 80,0 | 27,0 | 91,5 |
| Rendement en isomérisation (%) | 56,5 | 56,9 | 56,8 | 21,5 | 45,7 |
| Sélectivité en dismutation (%) | 7,0 | 6,8 | 6,8 | 55,0 | 5,5 |
| Sélectivité en craquage (%) | 1,5 | 1,6 | 1,6 | 1,7 | 0,8 |

On a obtenu sensiblement les mêmes résultats que ceux obtenus avec les catalyseurs A1, A2 et B en remplaçant le magnésium d'abord par du titane, puis du zinc et enfin de l'étain.

## Revendications

1. Catalyseur de type alumino-silicate contenant une mordénite caractérisé en ce qu' au moins un métal choisi parmi les métaux des groupes IIa, IVb, IIb et IVa de la classification périodique des éléments a été déposé sélectivement sur la surface extérieure des cristaux de la mordénite, ladite mordénite étant telle que :
   - son rapport atomique Si/Al global est compris entre 6 et 15,
   - sa teneur pondérale en sodium par rapport au poids de mordénite sèche est inférieure à 2000 ppm,
   - son volume de maille élémentaire est compris entre 2,725 et 2,785 $nm^3$,
   - sa capacité d'adsorption de n-hexane mesurée à 0°C sous une pression de 13 Torr ($1,7 \times 10^3$ Pa) est supérieure à 0,065 $cm^3$ liquide/gramme,
   - sa capacité d'adsorption d'isooctane mesurée à 40°C sous une pression de 26 Torr ($3,4 \times 10^3$ Pa) est inférieure à 0,068 $cm^3$ liquide/gramme.

2. Catalyseur selon la revendication 1 caractérisé en ce que ladite mordénite a une capacité d'adsorption de n-hexane mesurée à 0°C sous une pression de 13 Torr ($1,7 \times 10^3$ Pa) supérieure à 0,072 $cm^3$ liquide/gramme et une capacité d'adsorption d'isooctane mesurée à 40°C sous une pression de 26 Torr ($3,4 \times 10^3$ Pa) inférieure à 0,058 $cm^3$ liquide/gramme.

3. Catalyseur selon l'une des revendications 1 et 2 caractérisé en ce que la teneur pondérale de ladite mordénite en ledit métal est comprise entre 0,05 et 0,30 %.

4. Catalyseur selon l'une des revendications 1 à 3 caractérisé en ce que ledit métal est choisi parmi les métaux des groupes IIa et IIb de la classification périodique des éléments.

**5.** Catalyseur selon l'une des revendications 1 à 4 caractérisé en ce que ledit métal est le magnésium.

**6.** Catalyseur selon l'une des revendications 1 à 5 caractérisé en ce qu'il contient en outre au moins un métal du groupe VIII de la classification périodique des éléments.

**7.** Catalyseur selon la revendication 6 caractérisé en ce que ledit métal du groupe VIII est choisi dans le groupe formé par le platine et le palladium.

**8.** Catalyseur selon l'une des revendications 1 à 7 caractérisé en ce qu'il contient en outre une matrice.

**9.** Procédé de préparation d'une mordénite contenue dans un catalyseur selon l'une des revendications 1 à 8 à partir d'une mordénite forme H dans lequel le dépôt du métal choisi parmi les métaux des groupes IIa, IVb, IIb et IVa est réalisé par greffage sur la surface extérieure des cristaux de ladite mordénite forme H d'au moins un composé organométallique dudit métal.

**10.** Procédé de préparation d'une mordénite contenue dans un catalyseur selon l'une des revendications 6 à 8 à partir d'une mordénite forme H dans lequel le dépôt du métal choisi parmi les métaux des groupes IIa, IVb, IIb et IVa est réalisé par greffage d'au moins un composé organométallique dudit métal sur la surface extérieure des cristaux de ladite mordénite forme H après le dépôt préalable sur ladite mordénite d'au moins un métal du groupe VIII.

**11.** Procédé selon l'une des revendications 9 et 10 dans lequel ledit greffage a lieu en phase gazeuse, par sublimation dudit composé organométallique, à une température supérieure à 50°C.

**12.** Procédé selon l'une des revendications 9 et 10 dans lequel ledit greffage a lieu en phase liquide, ledit composé organométallique étant en solution dans un solvant, à une température supérieure à la température de congélation dudit solvant.

**13.** Procédé selon la revendication 11 caractérisé en ce qu'il comprend les étapes successives suivantes :
a) on sèche sous vide ou sous gaz inerte ladite mordénite entre 225 et 525°C pendant 2,5 à 5,5 heures,
b) on diminue la température sous vide ou sous gaz inerte jusqu'à une température comprise entre 100 et 200°C,
c) on met en contact ladite mordénite avec ledit composé organométallique à une température comprise entre 100 et 200°C pendant 10 à 60 minutes,
d) on élimine l'excès de composé organométallique par une purge sous vide ou sous gaz inerte,
e) on fait subir au solide obtenu à l'étape d) un traitement thermique en présence d'oxygène à une température supérieure à environ 250°C.

**14.** Procédé selon la revendication 12 caractérisé en ce qu'il comprend les étapes successives suivantes :
a) on sèche sous vide ou sous gaz inerte ladite mordénite entre 225 à 525°C pendant 2,5 à 5,5 heures,
b) on diminue la température sous vide ou sous gaz inerte jusqu'à une température comprise entre 5 et 35°C,
c) on met en contact sous vide ou sous gaz inerte ladite mordénite avec ledit composé organométallique en solution dans un solvant à une température comprise entre 5 et 35°C pendant 10 à 60 minutes,
d) on élimine la phase liquide sous vide ou sous gaz inerte,
e) on élimine l'excès de composé organométallique par un rinçage avec un solvant tel que celui utilisé à l'étape c),
f) on fait subir au solide obtenu à l'étape e) un traitement thermique en présence d'oxygène à une température supérieure à environ 250°C.

**15.** Procédé selon l'une des revendications 9 à 14 dans lequel ledit métal choisi parmi les métaux des groupes IIa, IVb, IIb et IVa est un métal des groupes IIa ou IIb.

**16.** Procédé selon l'une des revendications 9 à 14 dans lequel ledit métal choisi parmi les métaux des groupes IIa, IVb, IIb et IVa est le magnésium.

**17.** Procédé selon la revendication 16 dans lequel ledit composé organométallique est le bisnéopentylmagnésium $Mg(Np)_2$.

**18.** Utilisation d'un catalyseur selon l'une des revendications 1 à 8 ou contenant une mordénite préparée selon l'une des revendications 9 à 17 dans un procédé d'isomérisation d'une coupe C8 aromatique.

**Patentansprüche**

1.  Katalysator vom Aluminosilicat-Typ, der einen Mordenit enthält, dadurch gekennzeichnet, daß mindestens ein Metall, ausgewählt aus den Metallen der Gruppen IIa, IVb, IIb und IVa des Periodischen Systems der Elemente selektiv auf der äußeren Oberfläche der Kristalle des Mordenits abgelagert worden ist, wobei es sich bei dem genannten Mordenit um einen solchen handelt, dessen
    - Gesamtatomverhältnis Si/Al zwischen 6 und 15 liegt,
    - dessen Gewichtsgehalt an Natrium, bezogen auf das Gewicht des trockenen Mordenits, unter 2000 ppm liegt,
    - dessen Elementarzellen-Volumen zwischen 2725 und 2785 nm³ liegt,
    - dessen Adsorptionsvermögen für n-Hexan, gemessen bei 0°C unter einem Druck von 13 Torr (1,7 x 10³ Pa), oberhalb 0,065 cm³ Flüssigkeit/g liegt,
    - dessen Adsorptionsvermögen für Isooctan, gemessen bei 40°C unter einem Druck von 26 Torr (3,4 x 10³ Pa), unterhalb 0,068 cm³ Flüssigkeit/g liegt.

2.  Katalysator nach Anspruch 1, dadurch gekennzeichnet, daß der genannte Mordenit ein Adsorptionsvermögen für n-Hexan hat, das, gemessen bei 0°C unter einem Druck von 13 Torr (1,7 x 10³ Pa), oberhalb 0,072 cm³ Flüssigkeit/g liegt, und dessen Adsorptionsvermögen für Isooctan, gemessen bei 40°C unter einem Druck von 26 Torr (3,4 x 10³ Pa), unterhalb 0,058 cm³ Flüssigkeit/g liegt.

3.  Katalysator nach einem der Ansprüche 1 und 2, dadurch gekennzeichnet, daß der Gewichtsgehalt des genannten Mordenits an dem genannten Metall zwischen 0,05 und 0,30 % liegt.

4.  Katalysator nach einem der Ansprüche 1 bis 3, dadurch gekennzeichnet, daß das genannte Metall ausgewählt wird aus den Metallen der Gruppen IIa und IIb des Periodischen Systems der Elemente.

5.  Katalysator nach einem der Ansprüche 1 bis 4, dadurch gekennzeichnet, daß es sich bei dem genannten Metall um das Magnesium handelt.

6.  Katalysator nach einem der Ansprüche 1 bis 5, dadurch gekennzeichnet, daß er außerdem mindestens ein Metall der Gruppe VIII des Periodischen Systems der Elemente enthält.

7.  Katalysator nach Anspruch 6, dadurch gekennzeichnet, daß das genannte Metall der Gruppe VIII ausgewählt wird aus der Gruppe, die besteht aus Platin und Palladium.

8.  Katalysator nach einem der Ansprüche 1 bis 7, dadurch gekennzeichnet, daß er außerdem eine Matrix (einen Träger) enthält.

9.  Verfahren zur Herstellung eines Mordenits, wie er in einem Katalysator nach einem der Ansprüche 1 bis 8 enthalten ist, aus einem Mordenit in der H-Form, bei dem die Ablagerung des Metalls, ausgewählt aus den Metallen der Gruppen IIa, IVb, IIb und IVa erfolgt durch Aufpfropfen mindestens einer metallorganischen Verbindung des genannten Metalls auf die äußere Oberfläche der Kristalle des genannten Mordenits in der H-Form.

10. Verfahren zur Herstellung eines Mordenits, wie er in einem Katalysator nach einem der Ansprüche 6 bis 8 enthalten ist, aus einem Mordenit in der H-Form, bei dem die Ablagerung des Metalls, ausgewählt aus den Metallen der Gruppen IIa, IVb, IIb und IVa, erfolgt durch Aufpfropfen mindestens einer metallorganischen Verbindung des genannten Metalls auf die äußere Oberfläche der Kristalle des genannten Mordenits in der H-Form nach der vorherigen Ablagerung mindestens eines Metalls der Gruppe VIII auf dem genannten Mordenit.

11. Verfahren nach einem der Ansprüche 9 und 10, bei dem die Aufpfropfung in der Gasphase durch Sublimation der genannten metallorganischen Verbindung bei einer Temperatur oberhalb 50°C erfolgt.

12. Verfahren nach einem der Ansprüche 9 und 10, bei dem die genannte Aufpfropfung in flüssiger Phase in einer Lösung der genannten metallorganischen Verbindung in einem Lösungsmittel bei einer Temperatur oberhalb der Gefriertemperatur des Lösungsmittels erfolgt.

13. Verfahren nach Anspruch 11, dadurch gekennzeichnet, daß es die folgenden aufeinanderfolgenden Stufen umfaßt:

a) man trocknet den genannten Mordenit unter Vakuum oder unter einem Inertgas 2,5 bis 5,5 h lang zwischen 225 und 525°C,

b) man setzt die Temperatur unter Vakuum oder unter einem Inertgas bis auf einen Wert zwischen 100 und 200°C herab,

c) man bringt den genannten Mordenit mit der genannten metallorganischen Verbindung 10 bis 60 min lang bei einer Temperatur zwischen 100 und 200°C in Kontakt,

d) man eliminiert den Überschuß an metallorganischer Verbindung durch Ausblasen (Spülen) unter Vakuum oder unter einem Inertgas,

e) man unterwirft den in der Stufe (d) erhaltenen Feststoff in Gegenwart von Sauerstoff einer thermischen Behandlung bei einer Temperatur oberhalb etwa 250°C.

14. Verfahren nach Anspruch 12, dadurch gekennzeichnet, daß es die folgenden aufeinanderfolgenden Stufen umfaßt:

a) man trocknet den genannten Mordenit unter Vakuum oder unter einem Inertgas 2,5 bis 5,5 h lang zwischen 225 und 525°C,

b) man setzt die Temperatur unter Vakuum oder unter einem Inertgas bis auf einen Wert zwischen 5 und 35°C herab,

c) man bringt den genannten Mordenit unter Vakuum oder unter einem Inertgas mit der genannten metallorganischen Verbindung, gelöst in einem Lösungsmittel, 10 bis 60 min lang bei einer Temperatur zwischen 5 und 35°C in Kontakt,

d) man eliminiert die flüssige Phase unter Vakuum oder unter einem Inertgas,

e) man eliminiert den Überschuß an metallorganischer Verbindung durch Spülen mit einem Lösungsmittel, z.B. demjenigen, wie es in der Stufe (c) verwendet worden ist,

f) man unterwirft den in der Stufe (e) erhaltenen Feststoff in Gegenwart von Sauerstoff einer thermischen Behandlung bei einer Temperatur oberhalb etwa 250°C.

15. Verfahren nach einem der Ansprüche 9 bis 14, bei dem es sich bei dem Metall, das ausgewählt wird aus den Metallen der Gruppen IIa, IVb, IIb und IVa, um ein Metall der Gruppen IIa oder IIb handelt.

16. Verfahren nach einem der Ansprüche 9 bis 14, bei dem es sich bei dem genannten Metall, das ausgewählt wird aus den Metallen der Gruppen IIa, IVb, IIb und IVa, um das Magnesium handelt.

17. Verfahren nach Anspruch 16, bei dem es sich bei der genannten metallorganischen Verbindung um das Bisneopentylmagnesium Mg(Np)$_2$ handelt.

18. Verwendung eines Katalysators nach einem der Ansprüche 1 bis 8 oder eines solchen, der einen nach einem der Ansprüche 9 bis 10 hergestellten Mordenit enthält, in einem Verfahren zur Isomerisierung einer aromatischen C$_8$- Fraktion.

**Claims**

1. Catalyst of aluminosilicate type containing a mordenite characterized in that at least one metal selected from the metals of groups IIa, IVb, IIb and IVa of the periodic table has been deposited selectively on the outside surface of the crystals of the mordenite, said mordenite being such that:

-- its overall Si/Al atomic ratio is between 6 and 15,

-- its sodium content by weight relative to the weight of dry mordenite is less than 2000 ppm,

-- its elementary mesh volume is between 2.725 and 2.785 nm$^3$,

-- its n-hexane adsorption capacity measured at 0°C under a pressure of 13 torrs (1.7 x 10$^3$ Pa) is greater than 0.065 cm$^3$ of liquid/gram,

-- its isooctane adsorption capacity measured at 40°C under a pressure of 26 torrs (3.4 x 10$^3$ Pa) is less than 0.068 cm$^3$ of liquid/gram.

2. Catalyst according to claim 1, wherein said mordenite has an n-hexane adsorption capacity measured at 0°C under a pressure of 13 torrs (1.7 x 10$^3$ Pa) greater than 0.072 cm$^3$ of liquid/gram and an isooctane adsorption capacity measured at 40°C under a pressure of 26 torrs (3.4 x 10³ Pa) less than 0.058 cm³ of liquid/gram.

3. Catalyst according to one of claims 1 and 2, wherein the content by weight of said mordenite in said metal is between 0.05 and 0.30%.

4. Catalyst according to one of claims 1 to 3, wherein said metal is selected from the metals of groups IIa and IIb of the periodic table.

5. Catalyst according to one of claims 1 to 4, wherein said metal is magnesium.

6. Catalyst according to one of claims 1 to 5, wherein it further contains at least one metal of group VIII of the periodic table.

7. Catalyst according to claim 6, wherein said metal of group VIII is selected in the group formed by platinum and palladium.

8. Catalyst according to one of claims 1 to 7, wherein it further contains a matrix.

9. Process of preparation of a mordenite contained in a catalyst according to one of claims 1 to 8 starting from an H-shaped mordenite in which the deposition of the metal selected from the metals of groups IIa, IVb, IIb and IVa is performed by grafting on the outside surface of the crystals of said H-shaped mordenite of at least one organometallic compound of said metal.

10. Process of preparation of a mordenite contained in a catalyst according to one of claims 6 to 8 starting from an H-shaped mordenite in which the deposition of the metal selected from the metals of groups IIa, IVb, IIb and IVa is performed by grafting of at least one organometallic compound of said metal on the outside surface of the crystals of said H-shaped mordenite after the preliminary deposition on said mordenite of at least one metal of group VIII.

11. Process according to one of claims 9 and 10 in which said grafting takes place in gas phase, by sublimation of said organometallic compound, at a temperature greater than 50°C.

12. Process according to one of claims 9 and 10 in which said grafting takes place in liquid phase, said organometallic compound being in solution in a solvent, at a temperature greater than the freezing temperature of said solvent.

13. Process according to claim 11, wherein it comprises the following successive stages:
a) said mordenite is dried under vacuum or under inert gas between 225 and 525°C for 2.5 to 5.5 hours,
b) the temperature is reduced under vacuum or under inert gas to a temperature between 100 and 200°C,
c) said mordenite is put into contact with said organometallic compound at a temperature between 100 and 200°C for 10 to 60 minutes,
d) the organometallic compound excess is eliminated by a purging under vacuum or under inert gas,
e) the solid obtained in stage d) is put through a heat treatmment in the presence of oxygen at a temperature greater than about 250°C.

14. Process according to claim 12, wherein it comprises the following successive stages:
a) said mordenite is dried under vacuum or under inert gas between 225 and 525°C for 2.5 to 5.5 hours,
b) the temperature is reduced under vacuum or under inert gas to a temperature between 5 and 35°C,
c) said mordenite is put into contact under vacuum or under inert gas with said organometallic compound in solution in a solvent at a temperature between 5 and 35°C for 10 to 60 minutes,
d) the liquid phase under vacuum or under inert gas is eliminated,
e) the organometallic compound excess is eliminated by a rinsing with a solvent such as the one used in stage c),
f) the solid obtained in stage e) is put through a heat treatment in the presence of oxygen at a temperature greater than about 250°C.

15. Process according to one of claims 9 to 14, in which said metal selected from the metals of groups IIa, IVb, IIb and Iva is a metal of groups IIa or IIb.

16. Process according to one of claims 9 to 14 in which said metal selected from the metals of groups IIa, IVb, IIb and IVa is magnesium.

17. Process according to claim 16 in which said organometallic compound is bisneopentyl magnesium $Mg(Np)_2$.

18. Use of a catalyst according to one of claims 1 to 8 or containing a mordenite prepared according to one of claims 9 to 17 in an isomerization process of a C8 aromatic cut.